# FASCICULE DE BREVET EUROPEEN

(11) **EP 2 988 648 B1**
(45) Date de publication et mention de la délivrance du brevet: **10.05.2017**
(21) Numéro de dépôt: 14721919.0
(22) Date de dépôt: 10.04.2014
(51) Int. Cl.: A47L 15/00, A61C 19/00, A61B 90/00, F21V 33/00

(54) **DISPOSITIF DE LAVAGE D'ARTICLES**
VORRICHTUNG ZUM WASCHEN VON GEGENSTÄNDEN
DEVICE FOR WASHING OBJECTS

(30) Priorité: 25.04.2013 FR 1353792
(43) Date de publication de la demande: 02.03.2016
(73) Titulaire: Steris, 33185 Le Haillan (FR)
(72) Inventeur: L'HEGARAT, Jean-Marie, 33160 Saint Aubin de Medoc (FR); VATELOT, Charles, 33470 Le Teich (FR); OGER, Yvan, 33600 Pessac (FR); CHOROSY, Damien, 33185 Le Haillan (FR)
(74) Mandataire: Robert, Mathias
(86) Numéro de dépôt international: PCT/FR2014/050873
(87) Numéro de publication internationale: WO 2014/174177

(56) Documents cités:
- EP-A2- 1 933 094
- US-A1- 2004 264 168
- US-A1- 2009 272 136
- US-B2- 8 277 065

## Description

La présente invention concerne un dispositif de lavage d'articles, tels que des instruments ou dispositifs médicaux, dentaires, pharmaceutiques ou vétérinaires.

De tels articles sont exposés au sang ou à des fluides corporels nécessitant leur nettoyage et/ou leur désinfection. Pour cela, il est connu d'utiliser des dispositifs de lavage permettant de traiter ces instruments ou dispositifs entre chaque utilisation.

Les dispositifs de lavage permettent classiquement de réaliser une étape de lavage, une étape de rinçage et une étape de séchage des articles.

Le document WO 2009/058946 divulgue un dispositif de lavage de ce type, comprenant une cuve de lavage dans laquelle les articles à laver sont destinés à être insérés. La cuve comporte des parois latérales, une paroi supérieure et une paroi inférieure. Des balais d'aspersion tournants sont montés au centre des parois inférieure et supérieure. La cuve est destinée à contenir un support métallique sur lequel sont posés les articles ou des bacs contenant les articles.

Comme cela est connu en soi, deux des parois latérales de la cuve peuvent être formées par deux portes verticales opposées (« pass-through »), mobiles entre une position d'ouverture de la cuve et une position de fermeture de la cuve.

Dans un tel cas, les articles à laver sont introduits dans la cuve par l'une des portes, les articles lavés étant extraits de la cuve par l'autre porte, de façon à séparer les flux de circulation des articles. Une lampe est généralement encastrée dans un coin de la paroi supérieure. Cette lampe est protégée de l'eau ou de la vapeur circulant dans la cuve par une vitre.

Une fois que la cuve est pleine, c'est-à-dire que le support est engagé dans la cuve, la lampe située dans le coin de la cuve peine à éclairer de façon homogène l'ensemble de la cuve. Tout au plus, éclairant depuis l'intérieur, la lampe éclaire en direction de l'opérateur et l'éblouit. Par ailleurs, les dispositifs de lavage étant souvent nombreux et accolés les uns aux autres, il est difficile pour un opérateur situé à distance de visualiser l'état de fonctionnement de chacun d'entre eux.

Le document US 8277065 divulgue également un dispositif de lavage de l'art antérieur.

L'invention a notamment pour but d'apporter une solution simple, efficace et économique à ces problèmes.

A cet effet, elle propose un dispositif de lavage d'articles, comportant une cuve de lavage dans laquelle les articles à laver sont destinés à être insérés, ladite cuve étant délimitée par des parois dont au moins une comporte une zone transparente comprenant au moins deux parois transparentes écartées l'une de l'autre et délimitant entre elles un espace interne, caractérisé en ce que des moyens d'éclairage sont montés dans ledit espace interne et orientés de façon à éclairer au moins en partie la cuve.

De cette manière, les moyens d'éclairage peuvent éclairer la cuve de façon plus homogène, le contenu de cette dernière pouvant être visible par un opérateur, depuis l'extérieur, au travers de la zone transparente. Les moyens d'éclairage sont également protégés de l'eau circulant dans la cuve par la paroi transparente tournée du côté de la cuve.

De préférence, les moyens d'éclairage comportent des diodes électroluminescentes.

Dans ce cas, les moyens d'éclairage peuvent comporter au moins une bande support sur laquelle sont montées les diodes électroluminescentes.

En outre, la zone transparente peut comporter au moins un bord comportant un profilé de section en U, comprenant une base à partir de laquelle s'étendent un premier flanc situé du côté de la cuve et un second flanc opposé au premier flanc et de plus grande largeur que le premier flanc, la bande support étant fixée sur la face dudit second flanc tournée vers la cuve.

Les moyens d'éclairage peuvent également comporter des moyens de diffusion de la lumière, montés dans l'espace interne délimité entre les deux parois transparentes, de façon à améliorer encore l'homogénéité de l'éclairage.

Selon une forme de réalisation de l'invention, la zone transparente est de forme polygonale, par exemple de forme rectangulaire ou carrée, et comporte au moins deux bords opposés, par exemple deux bords verticaux, des moyens d'éclairage étant montés dans l'espace interne, au niveau de chacun des bords opposés.

Avantageusement, l'une au moins des parois de la cuve est formée par une porte mobile entre une position d'ouverture de la cuve et une position de fermeture de la cuve, ladite porte comportant la zone transparente équipée des moyens d'éclairage.

De préférence, deux parois opposées de la cuve sont formées par des portes mobiles, chaque porte comportant une zone transparente équipée des moyens d'éclairage.

Chaque porte peut comporter des moyens de détection de la position d'ouverture ou de fermeture, des moyens de détection d'un évènement et/ou des moyens de verrouillage de la porte en positon de fermeture, au moins un câble électrique étant relié ou alimentant lesdits moyens de détection, lesdits moyens de verrouillage et/ou lesdits moyens d'éclairage.

La porte peut par exemple être équipée d'un dispositif anti-pincement, apte à détecter un évènement tel que la présence d'une main d'un opérateur entre le bas de la porte et le bâti.

Dans ce cas, le câble électrique est supporté par une chaîne de guidage comportant des maillons articulés les uns par rapport aux autres et reliant la porte mobile au reste du dispositif.

Avantageusement, le dispositif de lavage peut comporter des moyens de pilotage des moyens d'éclairage, permettant de gérer la luminosité, le démarrage, l'arrêt et/ou la couleur des moyens d'éclairage.

De cette manière, il est possible d'éclairer différemment la cuve lors de chaque étape de lavage (pré-lavage, lavage, rinçage, désinfection, séchage), par exemple à l'aide de couleurs différentes.

L'invention sera mieux comprise et d'autres détails, caractéristiques et avantages de l'invention apparaîtront à la lecture de la description suivante faite à titre d'exemple non limitatif en référence aux dessins annexés dans lesquels :
- la figure 1 est une vue en perspective d'un dispositif d'un lavage selon l'invention, certains éléments ayant été retirés à des fins d'illustration,
- la figure 2 est une vue de face d'une porte du dispositif de la figure 1,
- la figure 3 est une vue éclatée, de face, de la porte de la figure 2,
- la figure 4 est une vue en coupe transversale de la porte des figures 2 et 3.

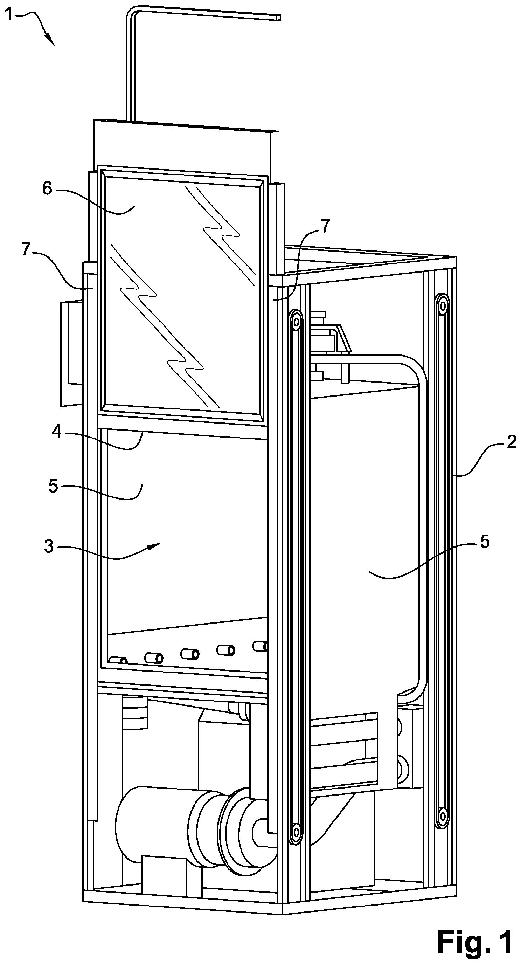

La figure 1 illustre un dispositif de lavage d'articles 1 selon une forme de réalisation de l'invention, comportant un bâti 2 comprenant une cuve parallélépipédique 3 délimitée par une paroi supérieure 4, une paroi inférieure (non représentée), deux parois latérales fixes et opposées 5 et deux parois latérales mobiles et opposées, formées par des portes 6 d'entrée et de sortie. Les portes 6 sont, dans cette forme de réalisation, montées coulissantes par rapport à des rails 7 du bâti 2, entre une position haute ou ouverte et une position basse ou fermée.

La cuve 3 est destinée à recevoir un support sur lequel sont posés les articles ou des bacs contenant les articles.

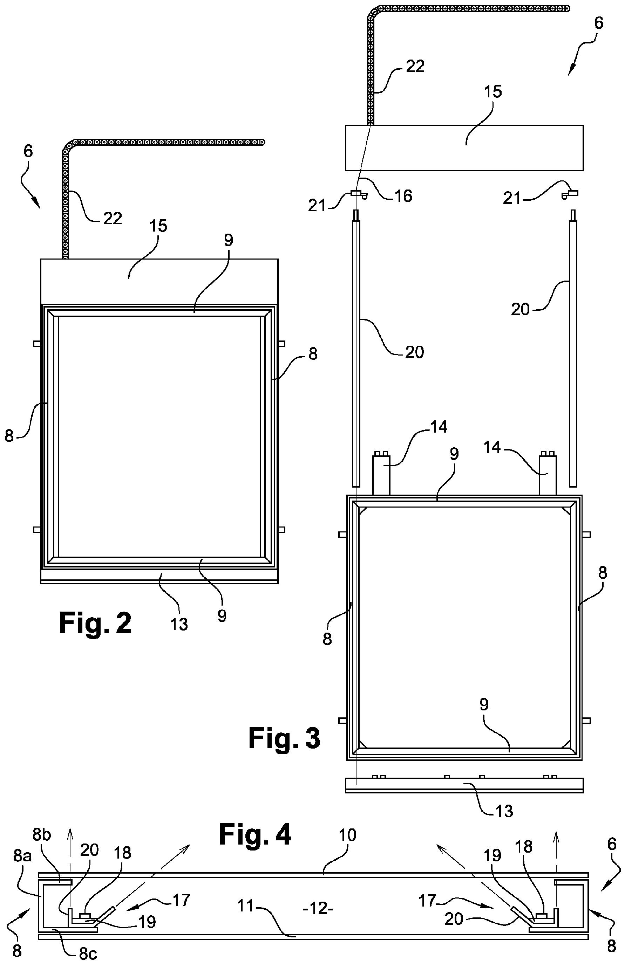

Comme cela est mieux visible aux figures 2 et 3 chaque porte 6 comporte un cadre rectangulaire délimitant une zone transparente, ledit cadre étant formé de profilés et comporte deux profilés ou bords verticaux 8 et deux profilés ou bords horizontaux 9.

Plus particulièrement, chaque profilé vertical 8 a une section en U (figure 4), comprenant une base 8a à partir de laquelle s'étendent un premier flanc 8b situé du côté de la cuve et un second flanc 8c opposé au premier flanc 8b et de plus grande largeur que le premier flanc 8b.

Les premiers flancs 8b des deux profilés verticaux 8 de chaque porte 6 servent à la fixation d'une première paroi transparente 10, telle qu'une vitre, tournée du côté de la cuve 3. Les seconds flancs 8c des profilés 8 précités servent à la fixation d'une seconde paroi transparente 11, telle qu'une vitre, tournée vers l'extérieur.

Les profilés horizontaux inférieur et supérieur 9 peuvent également présenter une structure en U et comporter des flancs servant à la fixation des parois transparentes 10, 11.

Ces parois 10, 11 peuvent être collées sur les flancs correspondants des profilés 8, 9.

Les deux parois 10, 11, parallèles l'une par rapport à l'autre, forment ainsi une zone transparente de type double-vitrage, entre lesquelles est formé un espace interne 12 délimité par les profilés horizontaux 9 et verticaux 8.

Un dispositif de détection 13 d'un évènement, tel que la présence d'une main entre la porte 6 et la cuve 3, est monté le long du profilé horizontal inférieur 9 du cadre. Un tel dispositif 13 est connu en soi.

La porte 6 comporte en outre deux plots 14 (figure 3) fixés sur le profilé horizontal supérieur 9, servant de butées afin de stopper le mouvement vertical de la porte.

Un bandeau 15 formé d'une tôle métallique s'étend également au-dessus du profilé horizontal supérieur 9, et recouvre les plots 14.

Un ou plusieurs câbles électriques 16 permettent d'alimenter et/ou de relier le dispositif de détection 13 et les capteurs précités à une unité d'alimentation et/ou de gestion des signaux issus des éléments précités (non représentée).

Conformément à l'invention, chaque porte 6 est équipée de moyens d'éclairage 17, logés dans l'espace interne 12 ménagé entre les parois transparentes 10, 11 correspondantes (figure 4).

Les moyens d'éclairage 17 comportent des diodes électroluminescentes 18, montées sur des bandes 19 fixées sur chaque profilé vertical 8 de la porte 6.

Plus particulièrement, chaque bande support 19 est fixée, par exemple par collage, sur un diffuseur vertical 20, lui-même appliqué sur la face du second flanc 8c correspondant, qui est tournée vers la cuve. Le diffuseur 20 a une section en forme général de U et être fixé, par ses extrémités, sur le profilé vertical correspondant 8 à l'aide de moyens de fixation de type clips 21 (figure 3).

Le diffuseur 20 et les diodes électroluminescentes 18 sont orientés de manière à éclairer la cuve 3 sur une plage angulaire importante, afin d'assurer un éclairage relativement homogène de la cuve 3. De plus, l'espace interne 12 étant étanche, les diodes électroluminescentes 18 sont protégées des fluides présents dans la cuve 3. La paroi transparente 10 tournée vers la cuve 3 et l'air ou le gaz contenu dans l'espace interne 12 permettent également d'isoler les diodes électroluminescentes 18 de la chaleur produite à l'intérieur de la cuve 3, en particulier lors de l'étape de séchage des articles.

Le ou les câbles électriques 16 précités permettent également d'alimenter les diodes électroluminescentes 18.

Ces câbles 16 sont supportés par une chaîne de guidage 22 comportant des maillons articulés les uns par rapport aux autres et reliant la porte mobile 6 au reste du dispositif 1.

## Revendications

1. Dispositif (1) de lavage d'articles, comportant une cuve (3) de lavage dans laquelle les articles à laver sont destinés à être insérés, ladite cuve (3) étant délimitée par des parois, l'une au moins des parois de la cuve étant formée par une porte (6) mobile entre une position d'ouverture de la cuve (3) et une position de fermeture de la cuve (3), ladite porte (6) comportant une zone transparente comprenant au moins deux parois transparentes (10, 11) écartées l'une de l'autre et délimitant entre elles un espace interne (12), des moyens d'éclairage (17) étant montés dans ledit espace interne (12) et orientés de façon à éclairer au moins en partie la cuve (3), aumoins un câble électrique (16) étant relié ou alimentant lesdits moyens d'éclairage (17), **caractérisé en ce que** ledit câble électrique est supporté par une chaîne de guidage (22) comportant des maillons articulés les uns par rapport aux autres et reliant la porte mobile (6) au reste du dispositif (1).

2. Dispositif (1) de lavage selon la revendication 1, **caractérisé en ce que** les moyens d'éclairage (17) comportent des diodes électroluminescentes (18).

3. Dispositif (1) de lavage selon la revendication 2, **caractérisé en ce que** les moyens d'éclairage (17) comportent au moins une bande support (19) sur laquelle sont montées les diodes électroluminescentes (18).

4. Dispositif (1) de lavage selon la revendication 3, **caractérisé en ce que** la zone transparente comporte au moins un bord comportant un profilé (8) de section en U, comprenant une base (8a) à partir de laquelle s'étendent un premier flanc (8b) situé du côté de la cuve (3) et un second flanc (8c) opposé au premier flanc (8b) et de plus grande largeur que le premier flanc (8b), la bande support (19) étant fixée sur la face dudit second flanc (8c) tournée vers la cuve (3).

5. Dispositif (1) de lavage selon l'une des revendications 1 à 4, **caractérisé en ce que** les moyens d'éclairage (17) comportent des moyens de diffusion de la lumière (20), montés dans l'espace interne (12) délimité entre les deux parois transparentes (10, 11).

6. Dispositif (1) de lavage selon l'une des revendications 1 à 5, **caractérisé en ce que** la zone transparente est de forme polygonale, par exemple de forme rectangulaire ou carrée, et comporte au moins deux bords opposés, par exemple deux bords verticaux (8), des moyens d'éclairage (17) étant montés dans l'espace interne (12), au niveau de chacun des bords opposés (8).

7. Dispositif (1) de lavage selon l'une des revendications précédentes, **caractérisé en ce que** deux parois opposées de la cuve sont formées par des portes mobiles (6), chaque porte (6) comportant une zone transparente équipée des moyens d'éclairage (17).

8. Dispositif (1) de lavage selon l'une des revendications précédentes, **caractérisé en ce que** chaque porte (6) comporte des moyens de détection de la position d'ouverture ou de fermeture , des moyens de détection d'un évènement (13) et/ou des moyens de verrouillage de la porte (6) en positon de fermeture, au moins un câble électrique (16) étant relié ou alimentant lesdits moyens de détection et/ou lesdits moyens de verrouillage.

9. Dispositif (1) de lavage selon l'une des revendications 1 à 8, **caractérisé en ce qu'**il comporte des moyens de pilotage des moyens d'éclairage (17), permettant de gérer la luminosité, le démarrage, l'arrêt et/ou la couleur des moyens d'éclairage (17).

## Patentansprüche

1. Vorrichtung (1) zum Waschen von Gegenständen, welche einen Waschbehälter (3) umfasst, in den die zu waschenden Gegenstände gelegt werden, wobei der Behälter (3) durch Wände abgegrenzt wird, und mindestens eine der Wände des Behälters durch eine Tür (6) geformt wird, die zwischen einer offenen Position des Behälters (3) und einer geschlossenen Position des Behälters (3) bewegt werden kann, wobei besagte Tür (6) einen durchsichtigen Bereich mit mindestens zwei durchsichtigen Wänden (10, 11) umfasst, die zueinander beabstandet sind und untereinander einen Innenraum (12) abgrenzen, wobei im Innenraum (12) Beleuchtungsmittel (17) montiert und so ausgerichtet sind, dass sie mindestens einen Teil des Behälters (3) beleuchten, wobei mindestens ein elektrisches Kabel (16) mit den Beleuchtungsmitteln (17) verbunden ist oder diese mit Strom versorgt, **dadurch gekennzeichnet, dass** das elektrische Kabel in einer Energieführungskette (22) bestehend aus gelenkig miteinander verbundenen Gliedern liegt und die bewegliche Tür (6) mit der übrigen Vorrichtung (1) verbindet.

2. Waschvorrichtung (1) nach Anspruch 1, **dadurch gekennzeichnet, dass** die Beleuchtungsmittel (17) Leuchtdioden (18) umfassen.

3. Waschvorrichtung (1) nach Anspruch 2, **dadurch gekennzeichnet, dass** die Beleuchtungsmittel (17) mindestens eine Stützleiste (19) umfassen, auf der die Leuchtdioden (18) montiert sind.

4. Waschvorrichtung (1) nach Anspruch 3, **dadurch gekennzeichnet, dass** der durchscheinende Bereich mindestens eine Kante mit einem Profil (8) mit u-förmigem Querschnitt aufweist, welches einen Sockel (8a) umfasst, von dem aus sich eine erste Flanke (8b) an der Seite des Behälters (3) und eine zweite Flanke (8c), welche länger ist als die erste Flanke (8b), gegenüber der ersten Flanke (8b) erstreckt, wobei die Stützleiste (19) an der Fläche der zweiten Flanke (8c) zum Behälter (3) hin gedreht befestigt ist.

5. Waschvorrichtung (1) nach einem der Ansprüche 1 bis 4, **dadurch gekennzeichnet, dass** die Beleuchtungsmittel (17) Mittel zur Streuung des Lichts (20) umfassen, die in den von den beiden durchscheinenden Wänden (10, 11) abgegrenzten Innenraum (12) montiert werden.

6. Waschvorrichtung (1) nach einem der Ansprüche 1 bis 5, **dadurch gekennzeichnet, dass** der durchscheinende Bereich eine polygonale Form, zum Beispiel eine rechteckige oder quadratische Form, hat und mindestens zwei einander gegenüber liegende Kanten, zum Beispiel zwei senkrechte Kanten (8), aufweist, wobei Beleuchtungsmittel (17) in Höhe der gegenüberliegenden Kanten (8) in den Innenbereich (12) montiert sind.

7. Waschvorrichtung (1) nach einem der vorhergehenden Ansprüche, **dadurch gekennzeichnet, dass** zwei einander gegenüberliegende Wände des Behälters durch bewegliche Türen (6) geformt werden, wobei jede Tür (6) einen durchscheinenden Bereich umfasst, der mit Beleuchtungsmitteln (17) ausgestattet ist.

8. Waschvorrichtung (1) nach einem der vorhergehenden Ansprüche, **dadurch gekennzeichnet, dass** jede Tür (6) Mittel zur Erkennung der offenen oder geschlossenen Position, Mittel zur Erkennung eines Ereignisses (13) und/oder Mittel zur Verriegelung der Tür (6) in der geschlossenen Position umfasst, wobei mindestens ein elektrisches Kabel (16) mit besagten Positionserfassungsmitteln und/oder Verriegelungsmitteln verbunden ist oder diese mit Strom versorgt.

9. Waschvorrichtung (1) nach einem der Ansprüche 1 bis 8, **dadurch gekennzeichnet, dass** sie Mittel zur Steuerung der Beleuchtungsmittel (17) umfasst, die das Steuern der Helligkeit, des Einschaltens, Ausschaltens und/oder der Farbe der Beleuchtungsmittel (17) ermöglichen.

## Claims

1. A device (1) for washing items, comprising a washing tank (3) where the items to be washed are intended to be inserted into, with said tank (3) being defined by walls, with at least one of the walls of the tank being formed by a door (6) which can move from one open position of the tank (3) to one closed position of the tank (3), with said door (6) comprising a transparent zone comprising at least two transparent walls (10, 11) spaced from one another and defining therebetween an internal space (12), with lighting means (17) being mounted in said internal space (12) and so oriented as to at least partially provide light to the tank (3), with at least one electric cable (16) being connected to or supplying said lighting means (17), **characterized in that** said electric cable is supported by a guiding chain (22) including mutually articulated links and connecting the mobile door (6) with the rest of the device (1).

2. A washing device (1) according to claim 1, **characterized in that** the lighting means (17) comprise light emitting diodes (18).

3. A washing device (1) according to claim 2, **characterized in that** the lighting means (17) comprise at least one supporting strip (19) whereon the light emitting diodes (18) are mounted.

4. A washing device (1) according to claim 3, **characterized in that** the transparent zone comprises at least one edge comprising a U-shaped section profile, comprising a base (8a) from which a first edge (8b) located on the tank (3) side and a second edge (8c) opposite the first edge (8b) and being wider than the first edge (8b) extend, with the supporting strip (19) being attached to the face of said second edge (8c) oriented towards the tank (3).

5. A washing device (1) according to one of claims 1 to 4, **characterized in that** the lighting means (17) comprise light diffusing means (20) mounted in the internal space (12) defined by the two transparent walls (10, 11).

6. A washing device (1) according to one of claims 1 to 5, **characterized in that** the transparent zone has a polygonal, for instance a rectangular or a square shape, and comprises at least two opposite edges, for instance two vertical edges (8), with lighting means (17) being mounted in the internal space (12), at each one of the opposite edges (8).

7. A washing device (1) according to one of the preceding claims, **characterized in that** two opposite walls of the tank are formed by mobile doors (6), with each door (6) comprising a transparent zone provided with lighting means (17).

8. A washing device (1) according to one of the preceding claims, **characterized in that** each door (6) comprises means for detecting the open or closed position, means for detecting an event (13) and/or means for locking the door (6) in the closed position, with at least one electric cable (16) being connected to or supplying said detection means and/or said locking means.

9. A washing device (1) according to one of claims 1 to 8, **characterized in that** it comprises means for controlling the lighting means (17) which make it possible to control luminosity, starting, stopping and/or colour of the lighting means (17).
